Europäisches Patentamt

European Patent Office

Office européen des brevets

(19)

(11) EP 0 648 469 B1

(12) FASCICULE DE BREVET EUROPEEN

(45) Date de publication et mention
de la délivrance du brevet:
**19.12.2001 Bulletin 2001/51**

(51) Int Cl.7: **A61B 8/00**, A61B 8/08

(21) Numéro de dépôt: **94202720.2**

(22) Date de dépôt: **22.09.1994**

(54) **Système et procédé de visualisation de données échographiques tridimensionnelles**

Vorrichtung und Verfahren zum Anzeigen von dreidimensionalen Daten eines
Ultraschallechographiegeräts

System and method for displaying 3-dimensional echographic data

(84) Etats contractants désignés:
**AT DE FR GB**

(30) Priorité: **29.09.1993 FR 9311609**

(43) Date de publication de la demande:
**19.04.1995 Bulletin 1995/16**

(73) Titulaire: **Koninklijke Philips Electronics N.V.
5621 BA Eindhoven (NL)**

(72) Inventeurs:
• **Collet-Billon, Antoine
F-75008 Paris (FR)**
• **Mallart, Raoul
F-75008 Paris (FR)**

(74) Mandataire: **Charpail, François et al
Société Civile S.P.I.D. 156, Boulevard
Haussmann
75008 Paris (FR)**

(56) Documents cités:
**EP-A- 0 531 081         WO-A-91/14400
US-A- 3 939 697         US-A- 4 821 731
US-A- 4 896 673         US-A- 5 197 476**

• **INNOVATION ET TECHNOLOGIE EN BIOLOGIE
ET MéDECINE, vol.13, no.1, 29 Février 1992,
PORTO (PORTUGAL) pages 117 - 125 A.
COLLET-BILLON ET AL. 'Imagerie
Echographique 3D'**

## Description

La présente invention concerne un système d'échographie tridimensionnelle (3D) comportant un échographe ultrasonore hôte muni d'un moniteur TV, une sonde ultrasonore à balayage 3D connectée audit échographe, pour l'analyse d'un sujet, des premiers moyens électroniques d'acquisition qui comportent une carte de contrôle en charge du balayage azimutal de la sonde et une carte mémoire stockant les lignes acoustiques en provenance de l'échographe, une station de travail constituée par un ensemble à microprocesseur muni d'une mémoire d'échographie pour le stockage de données échographiques 3D dudit sujet, la sonde, les premiers moyens électroniques et la station de travail étant reliés entre eux par un bus spécifique.

Plus généralement l'invention a pour objet un procédé d'échographie 3D consistant, en premier lieu, à faire l'acquisition de lignes échographiques distribuées dans un volume et passant par le point d'application d'une sonde échographique 3D contre un sujet, à convertir ces lignes sous forme d'éléments d'images volumétriques (voxels) numériques, repérés en coordonnées sphériques, puis à convertir les coordonnées desdits voxels en coordonnées cartésiennes.

L'échographie médicale traditionnelle consiste à former en temps réel, des images de structures biologiques (d'un sujet) en utilisant les ultrasons comme source d'information. Pour cela, on choisit comme plan de réception de l'écho un plan identique au plan d'émission, tout en illuminant le sujet choisi par un signal très bref. On bénéficie alors d'une information de distance pour localiser les inhomogénéités du sujet dans le plan d'émission-réception à travers le rayon et le centre de courbure du front d'onde réfléchi, mais aussi en tenant compte du temps τ d'arrivée du début de ce front d'onde au niveau du plan de réception :

$$\tau = 2z/C$$

z :   distance de la cible au plan d'émission-réception
C :   vitesse de propagation des ondes ultrasonores en milieu aqueux (1540 m/s).

On peut ainsi réaliser un tir, dans une direction en substance perpendiculaire au plan d'émission-réception. Pour réaliser une image, il suffit de répéter les tirs en déplaçant régulièrement la sonde pour obtenir l'information requise. De préférence, il est effectué un déplacement angulaire dans un plan, perpendiculaire au plan d'émission-réception qui constitue un plan de coupe du sujet, à visualiser, de façon à obtenir les données échographiques en coordonnées polaires, le nez de la sonde restant appliqué au même endroit, contre le sujet. Le balayage sectoriel dans le plan de coupe est généralement effectué électroniquement, à fréquence rapide pour que, à tout instant, l'on puisse afficher sur le moniteur TV associé à l'échographe le contenu échographique du plan de coupe visé par la sonde, c'est-à-dire le plan passant par l'axe de symétrie de la sonde qui est qualifiée de : bidimensionnelle (ou sonde 2D). L'utilisation, dans la sonde, de barrettes de transducteurs permet, par une commutation électronique rapide, d'atteindre une cadence d'images élevée, de l'ordre de 50 images par seconde, soit la possibilité d'imagerie en temps réel.

Partant de cet arrière plan technologique, lequel demeure conforme aux habitudes du praticien qui effectue toujours ainsi une analyse échographique en déplaçant la sonde contre le corps d'un patient, on sait maintenant faire de l'échographie tridimensionnelle qui, utilisant des sondes tridimensionnelles (ou 3D) permet l'acquisition rapide de données échographiques d'une partie de volume prédéterminée d'un sujet. Pour ce faire, est utilisée de préférence une sonde spécifique 3D constituée par des réseaux annulaires sectoriels bidimensionnels et comportant des moyens électroniques, respectivement mécaniques, pour effectuer un premier, respectivement un deuxième, balayages sectoriels croisés, la sonde pouvant émettre à une fréquence comprise entre 3 et 8 MHz.

Comme échographe à adapter à la sonde 3D pour l'acquisition des données volumétriques désormais repérées en coordonnées sphériques et non plus polaires, il est possible d'utiliser une extension d'un système 2D performant. Outre une ou plusieurs sondes 3D, ce système comporte en outre une électronique d'acquisition en charge du contrôle du balayage suivant la troisième dimension (azimutale) et de la localisation de la sonde dans cette direction, et une unité de contrôle du système et de traitement des données 3D. Cette dernière est une station de travail constituée par un microprocesseur muni de ses périphériques habituels. Sa tâche est d'une part le contrôle du système et d'autre part les post-traitements des données volumétriques, notamment une première opération qui consiste à effectuer une conversion de balayage en coordonnées cartésiennes des données 3D préalablement acquises en coordonnées sphériques.

Le matériel précité est décrit, avec plus de détails, dans la revue : Innovation et Technologie en Biologie et Médecine, vol.13, n° Spécial 1, 1992, dans l'article :

"Imagerie échographique 3D", pages 117 à 125, par MM. A. Collet-Billon, Y. Le Guérinel, F. Bernard, et J.M. Levaillant.

La procédure d'utilisation de ce matériel se déroule en phases successives :

Dans une première phase d'exploration, la sonde 3D est utilisée comme une sonde 2D classique, offrant les mêmes possibilités d'imagerie en temps réel sur l'écran du moniteur de l'échographe 2D-3D indiqué plus haut. L'utilisateur recherche la meilleure position de la sonde et contrôle le contenu du domaine exploré en modifiant l'incidence du plan de balayage à l'aide de boutons sur la sonde.

La phase d'acquisition qui suit est déclenchée en appuyant sur un autre bouton. La sonde balaye alors automatiquement le volume en quelques secondes. Pendant cette durée, tout mouvement de la sonde ou du patient doit être évité.

Après l'acquisition, les données sont transférées vers la station de travail en quelques secondes. L'utilisateur peut alors délimiter une région d'intérêt parmi les données acquises, avant de lancer la conversion de balayage, opération qui consiste à transformer la géométrie sphérique des lignes acoustiques en une géométrie cartésienne mieux adaptée à la visualisation.

Ensuite peut intervenir l'exploitation des données : l'utilisateur peut visualiser les données volumétriques via une interface utilisateur graphique entièrement contrôlable par une souris classique. Notamment, n'importe quelle coupe, d'orientation arbitraire, peut être obtenue simplement à partir de points de référence anatomiques (3 points) désignés à l'aide de la souris. Des vues dites cachées c'est-à-dire perpendiculaires à la direction du tir échographique, habituellement inaccessibles avec un échographe 2D, peuvent être facilement obtenues. Des séquences dynamiques de recoupes, d'avant en arrière ou en rotation, peuvent être également visualisées. Les vues multicoupes permettent l'inspection rapide du voisinage 3D de tout point du jeu de données. Dans une même séance, des images obtenues à partir de plusieurs jeux de données peuvent être affichées simultanément et comparées, etc... Toutes ces possibilités d'affichage, des rendus de volume en perspective, et d'autres encore sont à la portée de l'homme du métier, guidé par le praticien qui a, lui, une formation médicale. Cependant, il est aussi imaginable d'exploiter les données échographiques 3D contenues dans la station de travail d'une façon parfaitement adaptée à la pratique et aux habitudes de l'échographiste, et de procurer à ce dernier des moyens pour explorer lesdites données échographiques 3D en faisant exactement les mêmes gestes que pour une exploration échographique 2D en temps réel conventionnelle, ceci constituant l'idée de base de l'invention.

Sans préjudice du mode d'exploitation connu de données échographiques 3D décrit ci-dessus, l'invention vise à résoudre le problème technique qui consiste à pouvoir exploiter pleinement l'expérience et les compétences d'un praticien échographiste sans avoir à s'embarrasser du maniement d'une station de travail à laquelle il n'est pas nécessairement formé et au moyen de laquelle il obtiendrait une visualisation insuffisamment interactive ou à tout le moins peu familière.

Toujours dans le cadre de la visualisation de données échographiques 3D, un autre but de l'invention, qui détermine la principale application de cette dernière, est l'aide à l'apprentissage pour l'examen échographique 2D en temps réel, apprentissage qui est long et difficile car souvent sujet à de mauvaises interprétations. Comme cet apprentissage peut, selon l'invention, se faire en différé, hors de la présence d'un patient, il devient possible d'y consacrer beaucoup de temps, de faire des manipulations répétitives et comme les données 3D sont figées, de faire par exemple la recherche systématique de coupes qui seraient déterminées à l'avance par un praticien instructeur. De plus, plusieurs élèves peuvent opérer en même temps sur la même base de données 3D.

Le problème technique précité est résolu grâce au fait que le système d'échographie 3D défini au premier paragraphe est remarquable en ce qu'il comporte en outre, pour la lecture de ladite mémoire d'échographie, un dispositif de visualisation des données de ladite mémoire d'échographie constitué d'une part par un mannequin qui simule ledit sujet, d'autre part par un senseur d'orientation 3D manipulable comme un simulateur de sonde échographique bidimensionnelle (2D) pour pointer un plan de coupe dudit mannequin et connecté à la station de travail par l'intermédiaire d'un indicateur de coordonnées 3D, et des deuxièmes moyens électroniques d'affichage compris dans ladite station de travail, qui, à chaque position dudit senseur d'orientation 3 D font correspondre le plan de coupe pointé à un plan correspondant contenu sous forme d'éléments d'image volumétrique (voxels) dans la mémoire d'échographie, et qui affichent ce plan correspondant sur un écran dudit moniteur TV.

De même, le procédé d'échographie 3D défini au deuxième paragraphe est remarquable en ce que la visualisation desdits voxels en coordonnées cartésiennes, sous forme de plans de coupe, s'effectue par simulation de séance(s) d'échographie 2D, par manipulation conventionnelle d'un senseur d'orientation 3D, qui simule une sonde échographique 2D contre un mannequin qui simule ledit sujet, un rapport d'homothétie prédéterminé étant établi entre la taille du sujet et celle du mannequin, et l'adressage desdits voxels sous forme de plans de coupe à travers le volume que représentent lesdits voxels étant proportionné aux variations de déplacement et d'orientation dudit senseur contre ledit mannequin.

Le senseur d'orientation 3D, qui remplace ici la souris habituellement connectée à la station de travail, ou qui est reliée à la station de travail en complément de cette dernière au titre d'un périphérique supplémentaire, comprend de préférence trois bobines électromagnétiques qui détectent les champs magnétiques émis par un émetteur source disposé à proximité dudit mannequin, ledit senseur et ledit émetteur source étant connectés à un équipement électronique lui-même connecté à ladite station de travail.

Un tel senseur, précis à la fois en position spatiale et en position angulaire de précession, de nutation et de rotation propre, est fabriqué notamment par la société américaine Polhemus, P.O.Box 560, Colchester, Vermont 05446 ou encore par la société américaine Ascension Technology Corp. P.O. Box 527, Burlington, VT 05402.

La description qui suit en regard des dessins annexés, le tout donné à titre d'exemple, fera bien comprendre comment l'invention peut être réalisée.

La figure 1 représente un système d'échographie 3D de l'état de l'art.

La figure 2 montre le principe de base de l'invention.

La figure 3 est, pour l'essentiel, un schéma synoptique du système d'échographie 3D selon l'invention.

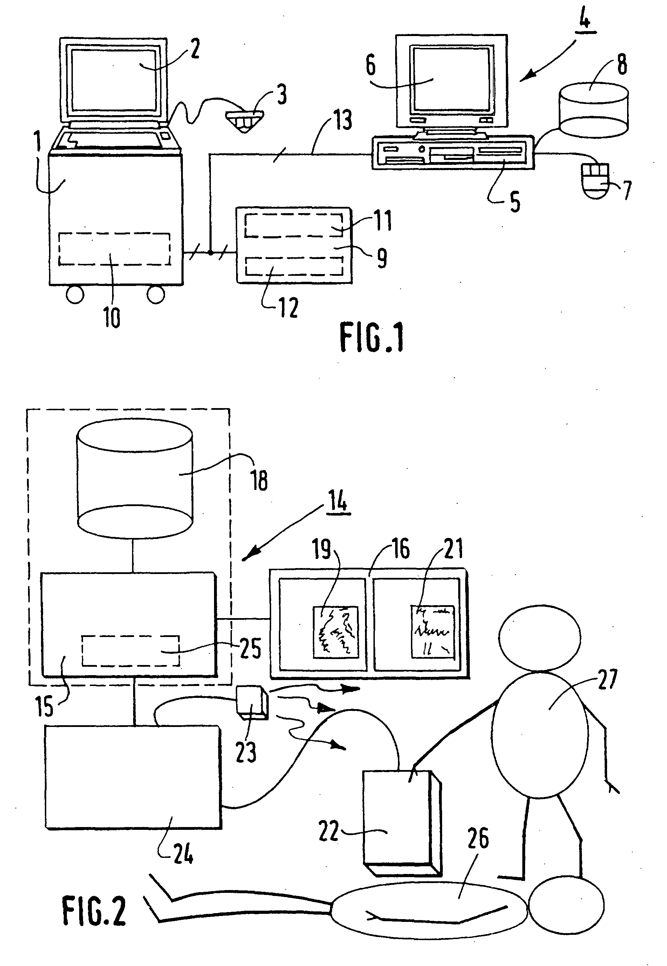

Le système de la figure 1 permet l'exploration de données échographiques distribuées dans un volume. Il est constitué par une extension d'un échographe 2D de haut de gamme. Ce système comporte un échographe hôte 3D, 1, qui est à l'origine un échographe 2D, muni d'un moniteur TV, 2, qui permet l'affichage en temps réel de coupes effectuées en échographie conventionnelle 2D au cours de l'analyse d'un sujet.

Pour l'adaptation au 3D, la sonde conventionnelle 2D est, en premier lieu, remplacée par une ou plusieurs sondes 3D, telles que 3, apte(s) à opérer un balayage additionnel en azimut, associée(s) à des moyens électroniques de commande (non représentés). Une station de travail 4, est constituée d'un microprocesseur 5 et de ses périphériques habituels, tel l'écran 6 et la souris 7. La station de travail retenue est une machine "SUN" par exemple la "SUN SPARC 2", avec une mémoire étendue symbolisée en 8, un large espace disque et une unité Exabyte. Sa tâche est, d'une part, le contrôle du système, d'autre part, les post-traitements des données volumétriques. De plus, un ensemble électronique d'acquisition 9 est aussi nécessaire, lors du passage de bidimensionnel en tridimensionnel, qui inclut une carte de contrôle 11 en charge du balayage additionnel et de l'enregistrement des positions azimutales de la sonde, ainsi qu'une carte mémoire 12 de stockage des lignes acoustiques démodulées et numérisées en provenance de l'échographe 1. Un bus spécifique 13, relie deux à deux de façon bidirectionnelle, les éléments 1, 9 et 4. Il s'agit par exemple d'un bus VME.

Les sondes 3D utilisées, telles que 3, sont de préférence des réseaux annulaires sectoriels 2D, extension des réseaux annulaires 1D classiques. Les réseaux annulaires offrent un avantage décisif par rapport aux autres sondes (barrettes comprises) : la possibilité de localisation- poursuite dans la direction hors plan, c'est-à-dire perpendiculaire au plan de l'image, soit la possibilité de contrôler l'épaisseur de coupe sur tout le champ exploré. Pour faciliter l'accès acoustique aux organes examinés, l'empreinte du nez de la sonde est aussi petite que possible ; son dessin minimise les réverbérations. En outre, en contact avec la peau du patient durant l'acquisition, qui se fait en coordonnées sphériques, le nez de la sonde reste immobile ; il n'y a donc pas de mouvement de tissus qui serait provoqué par le balayage. Trois boutons permettent de contrôler les phases successives d'une acquisition. De préférence, le balayage sectoriel 2D habituel s'effectue par des moyens électroniques, et le balayage sectoriel azimutal croisé, par des moyens mécaniques.

A l'ensemble électronique d'acquisition 9 il convient d'ajouter une carte esclave 10 insérée dans un panier de l'échographe 1, qui permet d'obtenir les lignes acoustiques immédiatement après qu'elles ont été numérisées, ainsi que les différents signaux de synchronisation nécessaires. Le balayage 2D manuel du réseau annulaire est pris en charge par l'échographe hôte.

La station de travail comporte un logiciel dédié, à la portée de l'informaticien moyen, qui est, de préférence, organisé en 5 modules : exploration-configuration, acquisition, conversion de balayage 3D, visualisation, archivage et gestion du système. La procédure d'utilisation du système de la figure 1 se déroule en phases successives qui sont déjà décrites ci-dessus avant l'énoncé des figures.

Concernant la phase d'acquisition, l'échographe fonctionne de façon séquentielle, c'est-à-dire qu'un tir acoustique n'est déclenché qu'après réception de la ligne acoustique précédente. Si l'on considère un jeu de données dont la densité de lignes est semblable dans le plan de l'image et en élévation, et d'ouverture angulaire identique dans ces 2 directions (environ 90°), le nombre total de lignes acoustiques est de l'ordre de 100 x 100. En supposant que la profondeur d'acquisition est 20 cm, le temps d'acquisition minimum est de 2,7 s, du fait de la vitesse du son dans les tissus (1540 m/s en moyenne). Les lignes acoustiques. numérisées, sont stockées dans la mémoire 12.

Après l'acquisition, une série d'images reconstruites à partir de données brutes est affichée sur l'écran 6 de la station de travail, afin de vérifier que la phase d'acquisition s'est correctement déroulée. Si tel est le cas, les données sont transférées de la mémoire 12 vers la station de travail en quelques secondes. Une région d'intérêt est alors sélectionnée dans les données acquises, puis est déclenchée, pour cette région, la conversion de balayage : coordonnées sphériques-coordonnées cartésiennes, pour les pixels volumétriques (ou voxels) sélectionnés et mémorisés en 4.

A ce stade, peut être effectuée l'exploitation des données échographiques, par tout moyen connu, notamment au moyen de la souris 7.

Le moyen le plus simple pour voir des données 3D sur un écran 2D consiste à offrir la possibilité d'afficher des coupes dans le volume acquis, suivant n'importe quelle orientation, et ce, en temps réel. Une coupe tomographique est en effet une image que l'échograhiste a l'habitude d'interpréter. Lorsqu'il utilise l'échographe, le médecin recherche les orientations de la sonde les plus favorables, c'est-à-dire celles qui contiennent des informations cliniques pertinentes ; ce mode d'utilisation n'est en pratique possible que parce que l'échographe offre une cadence d'image dite "temps réel", soit de 10 à 50 images par seconde.

Le système selon l'invention qui est décrit ci-dessous permet, notamment au médecin, d'effectuer un balayage à travers des données 3D préalablement acquises sur un patient virtuel, c'est-à-dire un mannequin ou un fantôme, de la même façon que s'il s'agissait d'un milieu-patient-réel.

La figure 2 permet d'expliciter le principe de base de l'invention. Une station de travail 14, comparable à la station 4 comporte un microprocesseur 15 avec une mémoire 18 et un écran 16. La mémoire 18 contient des données d'un sujet distribuées dans un volume (données 3D ou voxels), adressables en coordonnées cartésiennes, et dont on peut afficher des coupes 19, 21, sur l'écran 16, par tout moyen connu, notamment au moyen d'un clavier ou d'une souris (non représentés). Selon l'invention, pour l'affichage des coupes, on utilise, à titre de souris 3D, un dispositif de repérage à distance qui, dans le mode de réalisation de la figure 2, est constitué de trois éléments et dont l'élément essentiel est un senseur 3D, 22, qui fournit sa propre position dans l'espace (attitudes et coordonnées, soit trois angles non coplanaires et trois distances), avec précision et en temps réel, à raison de 50 positions par seconde. Le deuxième élément est un émetteur source 23 qui émet un champ (électro)magnétique capté par le senseur 22, les éléments 22 et 23 étant connectés à un coffret électronique 24, indicateur de position 3D (à 6 degrés de liberté), lui-même connecté au microprocesseur 15. En 15, une unité de traitement dédiée 25 a pour fonction d'effectuer un calcul de coupes, c'est-à-dire de déduire de chaque position sextuple fournie par le senseur 22 un plan de coupe correspondant, de façon bijective, pour ainsi dire à travers les voxels contenus en mémoire 18. Plus précisément, à partir d'un point de positionnement et d'une orientation initiaux choisis comme référence, au départ, un rapport d'homothétie constant est établi entre le déplacement du point de positionnement de la sonde et le déplacement du point de référence du plan de coupe correspondant et, de façon analogue, les variations d'orientation du senseur 22 dans l'espace sont fidèlement répercutées entre les plans de coupe correspondant à ces variations sur l'écran 16. L'unité de traitement 25 effectue donc, moyennant une programmation adéquate, laissée à l'initiative de l'homme de l'art, un calcul d'adressage particulier des voxels, en 18, visant à calquer le volume décrit par le senseur 22 en espace libre à proximité de l'émetteur-source 23 au volume mémorisé par les voxels en 18. Pour mieux concrétiser encore cet effet, et offrir ainsi un support physique aux manipulations du senseur, il est loisible d'encombrer l'espace libre en question d'un fantôme proportionné du sujet dont l'intérieur est imagé par les voxels de la mémoire 18. Si les voxels constituent des données échographiques 3D du corps d'un patient, le fantôme est alors un mannequin 26 qui représente ce patient (à l'échelle 1 pour un rapport d'homothétie de 1) et le senseur pourra être manipulé par un praticien 27 au même titre qu'une sonde ultrasonore échographique 2D. Avantageusement, le senseur peut être intégré à un boîtier, ou un simulateur de boîtier (non représentés) d'une telle sonde. On obtient ainsi une simulation complète d'une séance d'analyses échographiques 2D conventionnelles, à partir de données échographiques 3D figées, il est vrai, mais réelles. Il est également possible d'afficher simultanément et en temps réel plusieurs coupes, par exemple la coupe que donnerait la sonde utilisée de façon conventionnelle et une coupe dite C-scan, qui est orientée perpendiculairement au faisceau acoustique (à la ligne de tir échographique) et qui est impossible à obtenir avec un échographe conventionnel.

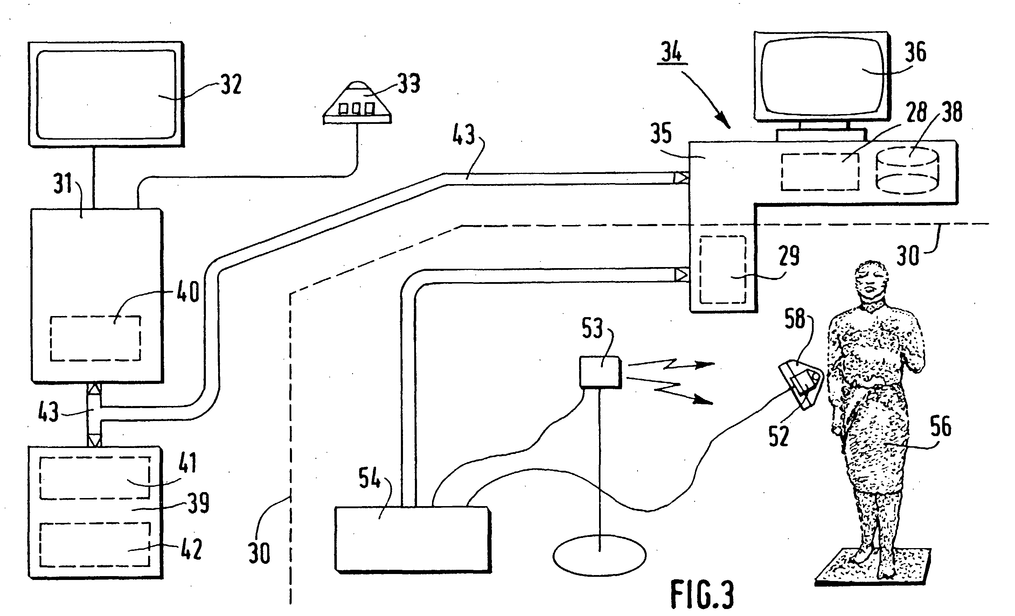

La figure 3 montre un mode de réalisation préféré de l'invention, appliqué à l'échographie ultrasonore, figure sur laquelle on retrouve la plupart des éléments (ou des équivalents) décrits ci-dessus séparément en référence aux figures 1 et 2. Sur cette figure, un échographe ultrasonore hôte 31 muni d'un moniteur TV 32 et occasionnellement d'une sonde 2D (non représentée) permet d'effectuer une analyse échographique 2D conventionnelle. Pour son adaptation à l'échographie 3D, il comporte en outre une sonde 3D, 33, de tout type connu, des moyens électroniques 40 (dits troisièmes moyens électroniques) pour fournir des lignes échographiques mesurées par la sonde 33. L'échographe 31 est en outre associé à des premiers moyens électroniques d'acquisition 39 qui comportent une carte de contrôle 41 équivalente à la carte de contrôle 11 de la figure 1 et une carte mémoire 42 équivalente à la carte mémoire 12 de la figure 1. Une station de travail 34, équivalente à 4, permet la gestion du système échographique 3D précité, un bus spécifique 43 reliant deux à deux et de façon bidirectionnelle les éléments 31, 39 et 34. La station de travail 34, comportant un microprocesseur 35 et un écran 36, est équivalente, quant à sa structure, aux stations de travail 4, de la figure 1, ou 14 de la figure 2 et ses fonctions constituent une hybridation des fonctions décrites pour 4, figure 1 et pour 14, figure 2. Le microprocesseur 35 comporte des moyens électroniques de conversion de balayage 28, dits quatrièmes moyens électroniques, une mémoire d'échographie 3D, 38, et des moyens électroniques d'affichage 29, dits deuxièmes moyens électroniques équivalents à l'unité de traitement 25 de la figure 2. Ces deuxièmes moyens électroniques ainsi que les autres éléments situés en dessous et à droite d'une ligne en trait interrompu 30 constituent le coeur de l'invention. Le senseur 3D, 52, l'émetteur source 53, l'indicateur de données 3D, 54, et le mannequin 56 sont équivalents aux éléments respectifs 22, 23, 24, 26 de la figure 2. La référence 58 désigne un boîtier ou un simulateur de boîtier de sonde échographique ultrasonore 2D.

Lorsqu'on souhaite faire, avec le système indiqué ci-dessus, une simulation d'analyse échographique 2D, une mise à l'origine du senseur 52 par rapport au mannequin 56 est nécessaire. En effet, un système d'axes orthonormé ox, oy, oz est lié au senseur, et un autre est lié à la source 53. Pour effectuer une mise à l'origine du senseur, on est amené à placer deux trièdres trirectangles, avec leurs axes homologues parallèles deux à deux, ce qui fournit le zéro pour les angles. Par ailleurs, à un plan de coupe prédéterminé pris comme origine, en mémoire 38, et affiché sur le moniteur 32, il faut associer le point correspondant avec l'orientation précitée, pour la position du senseur, par rapport au mannequin 56. (Il s'agit par exemple d'un plan qui passe au milieu des voxels, en 38, en suivant un axe préétabli, privilégié). En initialisant le système pour cette position d'origine, on obtient le zéro angulaire et spatial recherché. à partir duquel la simulation 2D en temps réel pourra être effectuée, tous les mouvements ultérieurs de la sonde étant ensuite mesurés par rapport à cette position de départ. Le degré de liberté supplémentaire qu'est le rapport d'homothétie variable offert par le système, comme déjà évoqué ci-dessus est, pour sa part, fixé au préalable, par une programmation (facile, pour l'homme du métier) avec, éventuellement, un étalonnage préalable, pour obtenir une coïncidence aussi proche que possible entre les voxels du mannequin visés par le senseur 52 et les voxels homologues contenus dans la mémoire 38, à partir des plans de coupe d'origine coïncidant lors de la mise à zéro.

L'invention n'a pas lieu de se limiter aux modes de réalisation décrits ci-dessus. En effet, les données 3D collectées en mémoire 8, 18 ou 38 pourraient être de toute autre nature que des données d'origine ultrasonore, par échographie. Il pourrait en particulier s'agir de voxels de synthèse représentant une image tridimensionnelle figurative ou pas. Le mannequin qui, dans tous les cas, doit donner une image visible de l'enveloppe du volume représenté par les voxels pourraît être en particulier un hologramme. Aussi, des senseurs avec leur électronique associée, autres que ceux décrits ci-dessus, sont utilisables, tels par exemple un senseur 3D à gyroscope, ou des dispositifs de repérage d'orientation basés sur le principe de retours d'ondes acoustiques sur des obstacles ou parois environnants captés par plusieurs micros, ou encore basés sur une triangulation obtenue à partir de plusieurs caméras qui filment en permanence le senseur 3D.

Sur le plan des applications, outre les applications à l'échographie 2D en temps réel décrites plus haut, l'invention présente un intérêt didactique, par exemple pour l'étude de l'anatomie humaine, animale ou de la botanique, ou pour la vulgarisation scientifique, en permettant de visualiser, simplement, la coupe d'une maquette vue sous n'importe quel angle.

### Revendications

1.  Système d'échographie tridimensionnelle (3D) comportant :

    -   un échographe ultrasonore hôte (31) muni d'un moniteur TV (32),

    -   une sonde ultrasonore à balayage 3D (33) connectée audit échographe pour l'analyse d'un sujet,
    -   des premiers moyens électroniques d'acquisition (39) qui comportent une carte de contrôle (41) en charge du balayage azimutal de la sonde et une carte mémoire (42) stockant les lignes acoustiques en provenance de l'échographe (31),
    -   une station de travail (34) constituée par un ensemble à microprocesseur (35) muni d'une mémoire d'échographie (38) pour le stockage de données échographiques 3D dudit sujet,

    la sonde (33), les premiers moyens électroniques (39) et la station de travail (34) étant reliés entre eux par un bus spécifique (43), système **caractérisé en ce qu'**il comporte en outre :

    -   pour la lecture de ladite mémoire d'échographie(38), un dispositif de visualisation des données de ladite mémoire d'échographie constitué d'une part par un mannequin (56) qui simule ledit sujet, d'autre part par un senseur d'orientation 3D (52) manipulable comme un simulateur de sonde échographique bidimensionnelle (2D) pour pointer un plan de coupe dudit mannequin et connecté à la station de travail (34) par l'intermédiaire d'un indicateur de coordonnées 3D (54),
    -   et des deuxièmes moyens électroniques d'affichage (29) compris dans ladite station de travail (34), qui, à chaque position dudit senseur d'orientation 3D (52) font correspondre le plan de coupe pointé à un plan correspondant contenu sous forme d'éléments d'image volumétrique (voxels) dans la mémoire d'échographie (38), et qui affichent ce plan correspondant sur un écran (36) dudit moniteur TV (32).

2.  Système d'échographie 3D selon la revendication 1 dans lequel ladite sonde ultrasonore à balayage 3D (33) est constituée par des réseaux annulaires sectoriels 2D et comporte des moyens électroniques, respectivement mécaniques, pour effectuer un premier, respectivement un deuxième, balayages sectoriels croisés, ladite sonde pouvant émettre à une fréquence comprise entre 3 et 8 MHz.

3.  Système d'échographie 3D selon la revendication 1 ou 2, dont ledit échographe hôte (31) est un échographe 2D qui comporte des troisièmes moyens électroniques (40) pour la fourniture des lignes acoustiques 3D après qu'elles ont été numérisées et des signaux de synchronisation, et dont ladite station de travail (34) comporte des quatrièmes moyens électroniques de conversion de balayage (28) en coordonnées cartésiennes desdites données échographiques 3D préalablement acquises en coordonnées sphériques.

4. Système d'échographie 3D selon l'une des revendications 1 à 3, **caractérisé en ce qu'**il comprend un émetteur source (53) disposé à proximité dudit mannequin (56), et **en ce que** ledit senseur d'orientation 3D (52) comprend trois bobines électromagnétiques pouvant détecter les champs magnétiques émis par ledit émetteur source, ledit senseur (52) et ledit émetteur source (53) étant connectés audit indicateur de coordonnées 3D (54).

5. Procédé d'échographie 3D consistant à faire l'acquisition de lignes échographiques distribuées dans un volume et passant par le point d'application d'une sonde échographique 3D (33) contre un sujet, à convertir ces lignes sous forme d'éléments d'images volumétriques (voxels) numériques, repérés en coordonnées sphériques, puis à convertir les coordonnées desdits voxels en coordonnées cartésiennes, **caractérisé en ce que** la visualisation desdits voxels en coordonnées cartésiennes, sous forme de plans de coupe, s'effectue par simulation de séance(s) d'échographie 2D, par manipulation conventionnelle d'un senseur d'orientation 3D (52) , qui simule une sonde échographique 2D contre un mannequin (56) qui simule ledit sujet, un rapport d'homothétie prédéterminé étant établi entre la taille du sujet et celle du mannequin, et l'adressage desdits voxels sous forme de plans de coupe à travers le volume que représentent lesdits voxels étant proportionné aux variations de déplacement et d'orientation dudit senseur (52) contre ledit mannequin (56).

6. Procédé d'échographie 3D selon la revendication 5, **caractérisé en ce que** ledit rapport d'homothétie est égal à 1.

7. Application du système d'échographie 3D selon l'une des revendications 1 à 4 ou du procédé d'échographie 3D selon la revendication 5 ou 6 à l'analyse échographique 2D différée d'une partie d'un sujet humain.

8. Application du système d'échographie 3D selon la revendication 7 à l'apprentissage de l'analyse échographique 2D en temps réel.

**Patentansprüche**

1. Dreidimensionale (3-D-)Echographievorrichtung mit:

   - einem Host-Ultraschallechographen (31), mit einem TV-Monitor (32) versehen,

   - einer 3-D-Ultraschall-Abtastsonde (33), an den besagten Echographen zur Analyse eines Subjektes angeschlossen,
   - ersten elektronischen Aufnahmemitteln (39) mit einer Steuerkarte (41) für die azimutale Abtastung der Sonde und einer Speicherkarte (42) zum Speichern der akustischen, aus dem Echographen (31) kommenden Linien,
   - einer Arbeitsstation (34), bestehend aus einem Mikroprozessorsatz (35), versehen mit einem Echographiespeicher (38) zum Speichern der echographischen 3-D-Daten des besagten Subjekts,

   wobei die Sonde (33), die ersten elektronischen Mittel (39) und die Arbeitsstation (34) untereinander über einen spezifischen Bus (43) verbunden sind, **dadurch gekennzeichnete** Vorrichtung, daß sie zusätzlich enthält:

   - zum Lesen des besagten Echographiespeichers (38), eine Vorrichtung zur Anzeige der Daten des besagten Echographiespeichers, gebildet einerseits aus einer Testpuppe (56) zur Simulation des besagten Subjektes, und andererseits aus einem 3-D-Orientierungssensor (52), wie eine zweidimensionale (2-D-)Echographiesonde zu handhaben, um eine Schnittebene der besagten Testpuppe zu markieren, über einen 3-D-Koordinatenindikator (54) mit der Arbeitsstation verbunden (34),
   - und zweite elektronische Anzeigemittel (29), in der besagten Arbeitsstation (34) enthalten, die an jeder Position des besagten 3-D-Orientierungssensors (52) die markierte Schnittebene mit einer entsprechenden Ebene verbinden, enthalten in der Form volumetrischer Bildelemente (Voxel) im Echographiespeicher (38), und die diese entsprechende Ebene auf dem Bildschirm 36 des besagten TV-Monitors (32) anzeigen.

2. 3-D-Echographievorrichtung nach Anspruch 1, in der die besagte 3-D-Ultraschall-Abtastsonde (33) aus 2-D-Ringabschnittnetzen gebildet wird, und die elektronische respektive mechanische Mittel enthält, um eine erste respektive eine zweite gekreuzte abschnittweise Abtastung vorzunehmen, wobei die Sonde mit einer zwischen 3 und 8 MHz enthaltenen Frequenz senden kann.

3. 3-D-Echographievorrichtung nach Anspruch 1 oder 2, wobei der besagte Host-Echograph (31) ein 2-D-Echograph ist, der dritte elektronische Mittel (40) enthält, um die akustischen 3-D-Linien zu liefern, nachdem sie digitalisiert wurden, und die Synchronisationssignale, und wobei die besagte Arbeitsstation (34) vierte elektronische Mittel enthält, zur Abtastkonvertierung (28) in logische Daten der besagten echographischen 3-D-Daten, zuvor in sphärischen Koordinaten erhalten.

4. 3-D-Echographievorrichtung nach einem der Ansprüche 1 bis 3, **dadurch gekennzeichnet, daß** sie einen Quellensender (53) enthält, in der Nähe der besagten Testpuppe (56) angeordnet, und daß der besagte 3-D-Orientierungssensor (52) drei elektromagnetische Spulen enthält, die die von dem besagten Quellensender ausgehenden magnetischen Felder erfassen können, wobei der besagte Sensor (52) und der besagte Quellensender (53) mit dem besagten 3-D-Koordinatenindikator (54) verbunden sind.

5. 3-D-Echographieverfahren, bestehend aus der Aufnahme echographischer Linien, in einem Volumen verteilt und durch den Anwendungspunkt einer echographischen 3-D-Sonde (33) gegen ein Subjekt verlaufend, der Konvertierung dieser Linien in der Form digitaler volumetrischer Bildelemente (Voxel), in sphärischen Koordinaten geortet, und dann der Konvertierung der Koordinaten der besagten Voxel in logische Koordinaten, **dadurch gekennzeichnet, daß** die Anzeige der besagen Voxel in logischen Koordinaten in der Form von Schnittebenen über die Simulation von 2-D-Echographiesitzung(en) verläuft, über herkömmliche Handhabung eines 3-D-Orientierungssensors (52), der eine echographische 2-D-Sonde gegen eine Testpuppe (56) simuliert, die das besagte Subjekt simuliert, wobei ein vorbestimmtes homothetisches Verhältnis zwischen der Größe des Subjektes und der der Testpuppe hergestellt wird, und die Adressierung der besagten Voxel in der Form von Schnittebenen über das Volumen, das die besagten Voxel darstellt, zu den Versetzungs- und Orientierungsvariationen des besagten Sensors (52) gegen die besagte Testpuppe (56) proportioniert wird.

6. 3-D-Echographieverfahren nach Anspruch 5, **dadurch gekennzeichnet, daß** das besagte homothetische Verhältnis gleich 1 ist.

7. Anwendung der 3-D-Echographievorrichtung nach einem der Ansprüche 1 bis 4 oder des 3-D-Echographieverfahrens nach dem Anspruch 5 oder 6 zur aufgezeichneten echographischen 2-D-Analyse eines Teils eines menschlichen Subjekts.

8. Anwendung der 3-D-Echographievorrichtung nach Anspruch 7 zum Erlernen der echographischen 2-D-Analyse in Echtzeit.

**Claims**

1. A three-dimensional (3D) echography system, including:

   - a host ultrasonic echograph (31) equipped with a TV monitor(32),
   - an ultrasonic probe with 3D scanning (33) connected to the said echograph, for analyzing a subject,
   - first electronic acquisition means (39) which include a control card (41) for taking charge of the azimuth scanning of the probe and a memory card (42) storing the acoustic lines originating from the echograph,
   - a workstation (34) consisting of a microprocessor assembly (35) equipped with an echography memory (38) for storing 3D echographic data on the said subject,
   - the probe (33), the first electronic means (39) and the workstation (34) being linked by a specific bus (43), which system is **characterized in that** it also includes:
   - for reading said echography memory (38), a device for visualizing the data in said echography memory, consisting, on the one hand, of a dummy (56) which simulates said subject, and, on the other hand, of a 3D orientation sensor (52) that can be manipulated like a two-dimensional (2D) echographic probe simulator so as to indicate a sectional plane of said dummy and is connected to the workstation (34), via a 3D coordinate indicator (54),
   - and second electronic display means (29) contained in said workstation (34), which, in each position of said 3D orientation sensor (52), make the indicated sectional plane correspond to an equivalent plane contained in the form of volumetric image elements (voxels) in the echography memory (38), and which display this plane on a screen (36) of said TV monitor (32).

2. A 3D echography system as claimed Claim 1, in which said ultrasonic probe (33) with 3D scanning consists of 2D, sectorial, annular arrays and includes electronic and mechanical means, respectively, for performing first and second crossed sectorial scans, respectively, said probe being capable of emitting at a frequency lying between 3 and 8 MHz.

3. A 3D echography system as claimed in Claim 1 or 2, in which said host echograph (31) is a 2D echograph which includes third electronic means (40) for supplying 3D acoustic lines after they have been digitized, and synchronization signals, and in which said workstation (34) includes fourth electronic means (28) for converting said 3D data previously acquired in spherical coordinates into a cartesian-coordinate scan.

4. A 3D echography system as claimed in one of the Claims 1 to 3, **characterized in that** it comprises a source emitter (53) arranged in the proximity of said dummy (56), and that said 3D orientation sensor (52)includes three electromagnetic coils which are capable of detecting the magnetic fields emitted by said source emitter, said sensor (52) and said source emitter (53) being connected to said 3D coordinate indicator (54).

5. A method of 3D echography, consisting in performing the acquisition of echographic lines distributed in a volume and passing through the point of application of a 3D echographic probe (33) to a subject, in converting these lines into the form of digital volumetric image elements (voxels), identified in spherical coordinates, and subsequently in converting the coordinates of said voxels into cartesian coordinates, **characterized in that** the visualization of said voxels in cartesian coordinates, in the form of sectional planes, takes place by simulation of a 2D echography session or sessions, by conventional manipulation of a 3D orientation sensor (52), which simulates a 2D echographic probe against a dummy (56) which simulates said subject, a predetermined homothetic ratio being established between the size of the subject and that of the dummy, and the addressing of said voxels in the form of sectional planes through the volume represented by said voxels, being proportional to the variations in displacement and in orientation of said sensor (52) against said dummy (56).

6. A method of 3D echography as claimed in Claim 5, **characterized in that** said homothetic ratio is equal to 1.

7. The application of the 3D echography system as claimed in one of the Claims 1 to 4 or of the 3D echography method as claimed in Claim 5 or 6 to the off-line 2D echography analysis of a part of a human subject.

8. The application of the 3D echography system as claimed in Claim 7 to the learning of 2D echography analysis in real time.

FIG.1

FIG.2

FIG.3

EP 0 648 469 B1